# EUROPEAN PATENT APPLICATION

(11) **EP 4 501 213 A1**
(43) Date of publication of application: **05.02.2025**
(21) Application number: 23780581.7
(22) Date of filing: 28.03.2023
(51) Int. Cl.: A61B 3/16

(54) **ULTRASONIC OPHTHALMIC TONOMETER**

(30) Priority: 30.03.2022 JP 2022056004
(71) Applicant: Nidek Co., Ltd., Gamagori-shi, Aichi, 443-0038 (JP)
(72) Inventor: UEMURA Tsutomu, Gamagori-shi Aichi 443-0038 (JP); HAMAGUCHI Koji, Gamagori-shi Aichi 443-0038 (JP); NAKAMURA Kenji, Gamagori-shi Aichi 443-0038 (JP)
(74) Representative: Hoefer & Partner Patentanwälte mbB
(86) International application number: PCT/JP2023/012629
(87) International publication number: WO 2023/190572

(57) **Abstract**

A technical object is to provide an ultrasonic ophthalmic tonometer capable of using a plurality of ultrasonic elements to irradiate a subject eye with an ultrasonic wave having a sufficient output for intraocular pressure measurement, and an ultrasonic ophthalmic tonometer control program. The ultrasonic ophthalmic tonometer measures an intraocular pressure of the subject eye by using the ultrasonic wave. The ultrasonic ophthalmic tonometer includes an imaging optical system that images the subject eye, and an irradiation unit including the plurality of ultrasonic elements and a support member supporting the ultrasonic elements, and irradiating the subject eye with the ultrasonic wave. An opening portion is provided with the support member. The opening portion allows an optical axis of the imaging optical system to pass therethrough.

## Description

### TECHNICAL FIELD

The present disclosure relates to an ultrasonic ophthalmic tonometer that measures an intraocular pressure of a subject eye by using an ultrasonic wave.

### BACKGROUND ART

A typical non-contact tonometer is an air-injection tonometer. The air-injection tonometer detects a deformation state of a cornea when air is injected onto the cornea and an air pressure injected onto the cornea, and converts the air pressure when the cornea is in an applanation state into an intraocular pressure.

As a non-contact tonometer, an ultrasonic ophthalmic tonometer that measures an intraocular pressure by using an ultrasonic wave has been proposed (see Patent Literature 1). The ultrasonic ophthalmic tonometer according to Patent Literature 1 detects a deformation state of a cornea when the ultrasonic wave is radiated to the cornea and a radiation pressure injected onto the cornea, and converts a radiation pressure in an applanation state into an intraocular pressure.

As an ultrasonic ophthalmic tonometer, a non-contact eyeball vibration type tonometer has been proposed which calculates an intraocular pressure of a subject eye based on vibration data of the subject eye in response to a sound wave from a parametric speaker in which a plurality of ultrasonic elements are used (see Patent Literature 2).

### CITATION LIST

### PATENT LITERATURE

Patent Literature 1: JPH05-253190A
Patent Literature 2: JP2015-092980A

### SUMMARY OF INVENTION

In a case in which a plurality of ultrasonic elements are used, when a parametric speaker is disposed obliquely to a subject eye as in Patent Literature 2 in order to dispose an observation optical system for observing an anterior segment of the subject eye, an acoustic radiation pressure (or an acoustic pressure) high enough to measure an intraocular pressure cannot be generated by deforming a cornea into a predetermined state (for example, an applanation state) as in Patent Literature 1.

In view of the related problem, a technical object of the present disclosure is to provide an ultrasonic ophthalmic tonometer capable of using a plurality of ultrasonic elements to irradiate a subject eye with an ultrasonic wave having a sufficient output for intraocular pressure measurement.

In order to solve the above problem, the present disclosure has the following configuration.
(1) An ultrasonic ophthalmic tonometer that measures an intraocular pressure of a subject eye by using an ultrasonic wave, the ultrasonic ophthalmic tonometer including: an imaging optical system configured to image the subject eye; and an irradiation unit including a plurality of ultrasonic elements and a support member configured to support the ultrasonic elements and irradiating the subject eye with an ultrasonic wave, in which an opening portion is provided with the support member, the opening portion configured to allow an optical axis of the imaging optical system to pass therethrough.

### BRIEF DESCRIPTION OF DRAWINGS

[FIG. 1] FIG. 1 is an external view of an ultrasonic ophthalmic tonometer.
[FIG. 2] FIG. 2 is a schematic view illustrating the inside of a housing.
[FIG. 3] FIG. 3 is a schematic view illustrating a configuration of an irradiation unit.
[FIG. 4] FIG. 4 is a block diagram illustrating a control system.
[FIG. 5] FIG. 5 is a flowchart illustrating a measurement operation.
[FIG. 6] FIG. 6 is a diagram illustrating a modification of the irradiation unit.
[FIG. 7] FIG. 7 is a diagram illustrating a modification of the irradiation unit.

### DESCRIPTION OF EMBODIMENTS

### <Embodiment>

Hereinafter, an embodiment according to the present disclosure will be described. The ultrasonic ophthalmic tonometer according to the present embodiment measures an intraocular pressure of a subject eye in a non-contact manner by using, for example, an ultrasonic wave. The ultrasonic ophthalmic tonometer measures the intraocular pressure by, for example, optically or acoustically detecting a shape change or vibration of the subject eye when the subject eye is irradiated with the ultrasonic wave. For example, the ultrasonic ophthalmic tonometer continuously irradiates a cornea with pulse waves or burst waves, and calculates the intraocular pressure based on output information of the ultrasonic wave when the cornea is deformed into a predetermined state (for example, an applanation state or a flat state). The output information includes, for example, an acoustic pressure, an acoustic radiation pressure, an irradiation time (for example, an elapsed time after a trigger signal is input), and a frequency of the ultrasonic wave. When the cornea of the subject eye is deformed, for example, the acoustic pressure, the acoustic radiation pressure, and an acoustic flow the ultrasonic wave are used.

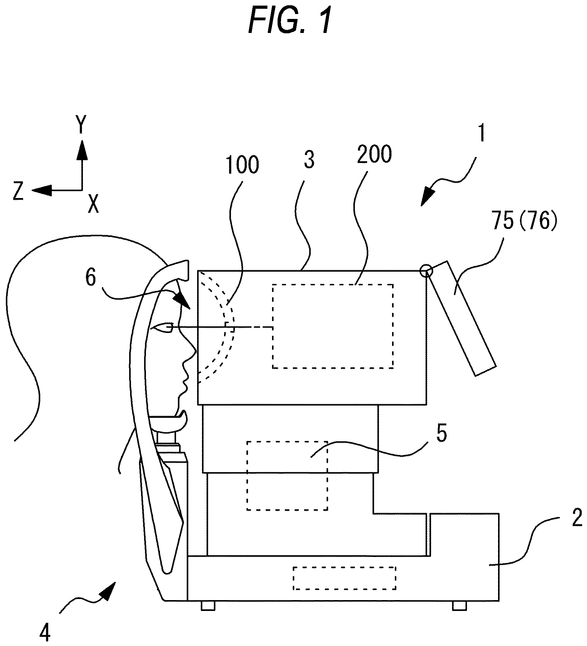

FIG. 1 illustrates an appearance of a device. An ultrasonic ophthalmic tonometer 1 includes, for example, a base 2, a housing 3, a face support unit 4, and a drive unit 5. An irradiation unit 100, an optical system 200, and the like to be described later are disposed inside the housing 3. The face support unit 4 supports a face of a subject person. The face support unit 4 is installed, for example, on the base 2. The drive unit 5 moves the housing 3 with respect to the base 2 for alignment, for example.

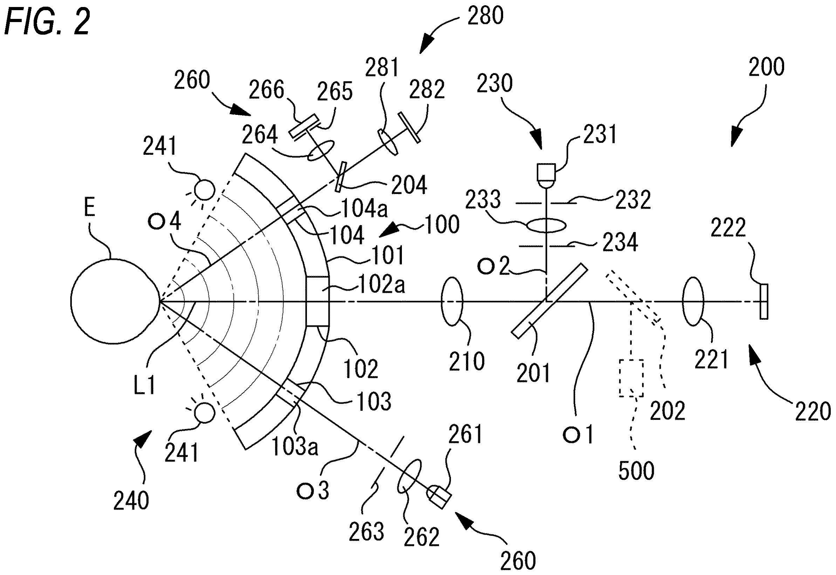

FIG. 2 is a schematic view illustrating a main configuration inside the housing. For example, the irradiation unit 100, the optical system 200 are disposed inside the housing 3. The irradiation unit 100 and the optical system 200 will be described in this order with reference to FIG. 2.

### <Irradiation Unit>

The irradiation unit 100 irradiates a subject eye E with an ultrasonic wave, for example. For example, the irradiation unit 100 irradiates the cornea with the ultrasonic wave to generate an acoustic radiation pressure on the cornea. The acoustic radiation pressure is, for example, a force acting in a traveling direction of the sound wave. The ultrasonic ophthalmic tonometer 1 according to the present embodiment deforms the cornea by using the acoustic radiation pressure, for example.

The irradiation unit 100 according to the present embodiment is a parametric speaker, and is provided with a plurality of (for example, two or more) ultrasonic elements 110. The irradiation unit 100 is disposed in a front direction of the subject eye. The ultrasonic element 110 generates the ultrasonic wave. The ultrasonic element 110 may be a piezoelectric element (for example, a piezoelectric ceramic), a magnetostrictive element, or the like. The plurality of ultrasonic elements 110 are supported by, for example, a support member 101. The support member 101 according to the present embodiment has a hemispherical shape (a bowl shape), and the respective ultrasonic elements 110 are arranged in a spherical shape by being supported by the support member 101. Accordingly, acoustic axes of the respective ultrasonic elements 110 intersect at one point, and the ultrasonic wave is easily focused. However, the ultrasonic elements 110 may not necessarily be arranged in a spherical shape, may be arranged in an aspherical (curved) shape such as an oval spherical surface, or may be arranged in a planar shape.

The support member 101 is provided with an opening portion 102 that allows an optical axis of the optical system 200 to pass therethrough. In the present embodiment, the opening portion 102 that allows an optical axis O1 of an observation system (an imaging optical system) 220 to be described later to pass therethrough is provided at a center of the support member 101. That is, the optical axis O1 of the observation system 220 is disposed in the opening portion 102. In this way, by providing the opening portion 102 that allows the optical axis O1 of the observation system 220 to pass therethrough in the support member 101 supporting the respective ultrasonic elements 110, it is possible to observe the subject eye while emitting the ultrasonic wave having a sufficient output. In the present embodiment, a hole 103 (a first hole) that allows a light projecting optical axis O3 of a deformation detection system 260 (or a Z alignment detection system 280) to pass therethrough and a hole 104 (a second hole) that allows a light receiving optical axis O4 to pass therethrough are provided on the left and right of the opening portion 102. Optical members such as transparent plates (or lenses) 102a, 103a, and 104a may be disposed in the opening portion 102 and the holes 103 and 104. Accordingly, foreign matters (such as dust) can be prevented from entering the device through the opening portion 102 and the holes 103 and 104.

The irradiation unit 100 according to the present embodiment is disposed in a manner that a sound source area increases. That is, the ultrasonic elements 110 according to the present embodiment are arranged in a manner that a density of the ultrasonic elements 110 increases. A magnitude of the acoustic radiation pressure (or the acoustic pressure) is determined by an acoustic intensity to be supplied from a sound source and a spot diameter, and in particular, the larger the sound source area, the greater the acoustic intensity to be supplied and the greater the acoustic radiation pressure to be generated. Therefore, by increasing an arrangement density of the ultrasonic elements 110 as in the present embodiment, the acoustic radiation pressure (or the acoustic pressure) can be increased while reducing a size of the irradiation unit 100.

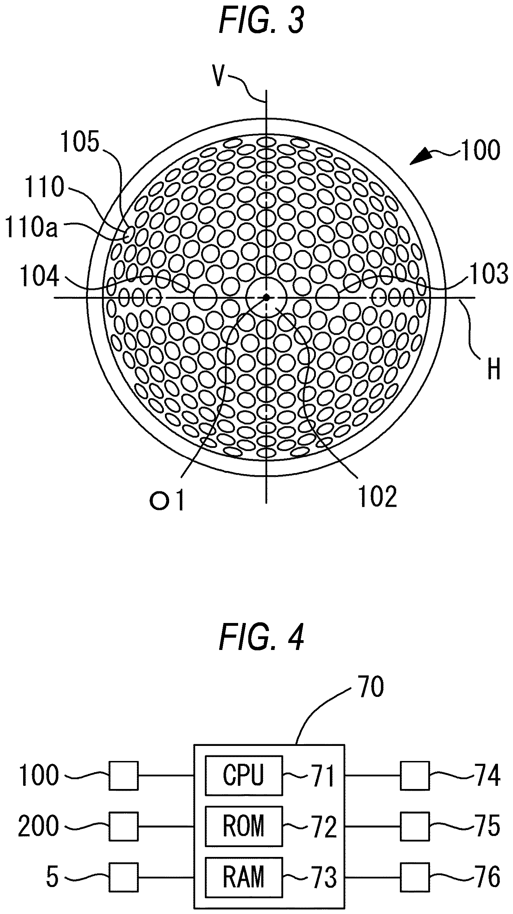

FIG. 3 is a view of the irradiation unit 100 as viewed from a subject side. As illustrated in FIG. 3, the support member 101 is provided with a plurality of installation holes 105 for installing the ultrasonic elements 110 around the opening portion 102. The ultrasonic element 110 is disposed in a manner of surrounding the opening portion 102 that allows the optical axis O1 of the observation system 220 to pass therethrough by being installed in the installation hole 105.

In the irradiation unit 100 according to the present embodiment, a size of the ultrasonic element 110 is reduced, and a large number of ultrasonic elements 110 are arranged, thereby reducing gaps between the ultrasonic elements, increasing the sound source area. A diameter of the ultrasonic element 110 according to the present embodiment (for example, a diameter in a direction perpendicular to the acoustic axis of the ultrasonic element 110) is smaller than a diameter of the opening portion 102 for the observation system 220 (for example, a diameter in a direction perpendicular to the optical axis O1). Accordingly, the gap between the ultrasonic elements when the ultrasonic elements 110 are arranged around the opening portion 102 can be reduced, and the density of the ultrasonic elements 110 in the parametric speaker can be increased. For example, the opening portion 102 for the observation system 220 according to the present embodiment has a diameter of Φ20 mm, and the ultrasonic element 110 has a diameter of Φ15 mm or less.

A distance between the ultrasonic elements (a distance between centers) may be equal to or less than a wavelength of the ultrasonic wave to be output. Accordingly, generation of grating lobes can be prevented. The grating lobe is unnecessary ultrasonic radiation generated when the distance between centers of the ultrasonic elements exceeds the wavelength of the ultrasonic wave. For example, when a frequency of the ultrasonic wave to be output is 40 kHz, the wavelength of the ultrasonic wave is 8.5 mm, and it is advisable to set the distance between centers of the ultrasonic elements to 8.5 mm or less.

The ultrasonic elements 110 according to the present embodiment are arranged in a circular shape when viewed from a direction of the subject eye. Accordingly, when the cornea of the subject eye is in an applanation state, a circular applanation plane can be suitably formed. For example, when determining an intraocular pressure value based on a diameter of the applanation plane as in a goldmann tonometer, a measurement accuracy can be improved by forming a circular applanation plane. The ultrasonic elements 110 may be concentrically arranged.

As illustrated in FIG. 3, the respective ultrasonic elements 110 may be arranged in a manner that sound source areas (a total area of irradiation surfaces 110a of the respective ultrasonic elements 110) are symmetrical (the same) in upper and lower regions when the support member 101 is divided by a horizontal plane H including the optical axis O1, or may be arranged in a manner that the sound source areas are symmetrical (the same) in left and right regions when the support member 101 is divided by a vertical plane V including the optical axis O1. Accordingly, deflection of an acoustic axis L1 of the ultrasonic wave with respect to the optical axis O1 can be prevented. By arranging the sound source areas symmetrically in the left and right, occurrence of a difference in measurement conditions can be prevented between a right eye and a left eye. Of course, the respective ultrasonic elements 110 may be arranged to be plane-symmetric (vertically symmetric) with respect to the horizontal plane H, or the respective ultrasonic elements 110 may be arranged to be plane-symmetric (left-right symmetric) with respect to the vertical plane V The acoustic axis is, for example, a central axis of the ultrasonic wave emitted by the irradiation unit 100. For example, the acoustic axis extends in the traveling direction of the ultrasonic wave, or in a vibration direction of the irradiation unit 100, and passes through a focal position where the ultrasonic wave emitted from the irradiation unit 100 are focused.

As illustrated in FIG. 3, the ultrasonic elements 110 may be arranged in a manner that the ultrasonic elements 110 adjacent to each other in a left-right direction have different heights. That is, by making the height of the ultrasonic elements 110 different between any row of ultrasonic elements 110 and an adjacent row, an interval between vertical rows of ultrasonic elements 110 can be narrowed. Accordingly, for example, wasted space can be reduced as compared with a case in which the ultrasonic elements 110 are arranged in a grid pattern at regular intervals in vertical and horizontal directions.

The ultrasonic elements 110 according to the present embodiment are arranged in a manner as described with reference to FIG. 3, so that the arrangement density is increased and the sound source area is increased. Accordingly, the acoustic radiation pressure (or the acoustic pressure) can be increased while reducing the size of the irradiation unit 100. However, the arrangement of the ultrasonic elements 110 is not necessarily the same as that illustrated in FIG. 3. For example, the arrangement density can be improved by taking at least one of elements such as the size, the interval, the arrangement shape, the symmetry, and the height of each ultrasonic element 110 into the arrangement.

### <Optical System>

The optical system 200, for example, observes and measures the subject eye (see FIG. 2). The optical system 200 includes, for example, an objective system 210, the observation system 220, a fixation target projection system 230, the deformation detection system 260, a dichroic mirror 201, and a beam splitter 204.

The objective system 210 is, for example, an optical system for taking light from the outside of the housing 3 into the optical system 200 or for emitting light from the optical system 200 to the outside of the housing 3. The objective system 210 includes, for example, an optical element. The objective system 210 may include an optical element such as an objective lens and a relay lens.

An illumination system 240 illuminates the subject eye. The illumination system 240 illuminates the subject eye with, for example, infrared light. The illumination system 240 includes, for example, an illumination light source 241. The illumination light source 241 is disposed, for example, obliquely forward of the subject eye. The illumination light source 241 emits, for example, infrared light. The illumination system 240 may include a plurality of illumination light sources 241.

The observation system 220 images an observation image of the subject eye, for example. The observation system 220 images an anterior segment image of the subject eye, for example. The observation system 220 includes, for example, a light receiving lens 221, and a light receiving element 222. The observation system 220 receives, for example, light from the illumination light source 241 reflected by the subject eye. The observation system 220 receives, for example, a reflected light beam from the subject eye centered on the optical axis O1. For example, light reflected from the subject eye passes through the opening portion 102 of the irradiation unit 100 and is received by the light receiving element 222 via the objective system 210 and the light receiving lens 221. A corneal reflection bright point of the illumination light source 241 received by the light receiving element 222 is used, for example, for alignment (XY alignment) in the vertical and horizontal directions. In this case, for example, the illumination system 240 and the observation system 220 function as an XY alignment detection unit. Of course, in addition to the illumination system 240, an index projection system that projects an index for the XY alignment from the optical axis O1 onto the subject eye may be provided. In this case, since a corneal central bright point appears in the observation image of the observation system 220, the XY alignment may be performed based on the corneal central bright point.

The fixation target projection system 230 projects a fixation target onto the subject eye, for example. The fixation target projection system 230 includes, for example, a target light source 231, an aperture 232, a light projecting lens 233, and an aperture 234. Light from the target light source 231 passes through the aperture 232, the light projecting lens 233, the aperture 234, and the like along the optical axis 02, and is reflected by the dichroic mirror 201. The dichroic mirror 201, for example, makes the optical axis O2 of the fixation target projection system 230 coaxial with the optical axis O1. Light from the target light source 231 reflected by the dichroic mirror 201 passes through the objective system 210 along the optical axis O1 and is emitted onto the subject eye. When a target of the fixation target projection system 230 is fixated by the subject, a line of sight of the subject is stabilized.

The deformation detection system 260 detects, for example, deformation of the cornea of the subject eye. The deformation detection system 260 includes, for example, a light source 261, a light projecting lens 262, an aperture 263, a light receiving lens 264, an aperture 265, and a light receiving element 266. Light from the light source 261 passes through the light projecting lens 262 and the aperture 263 along the optical axis O3, and is emitted onto the subject eye, for example. Then, reflected light reflected by the subject eye is reflected by the beam splitter 204 along the optical axis O4, and then passes through the light receiving lens 264 and the aperture 265 and is received by the light receiving element 266. For example, the deformation detection system 260 may detect the deformation of the cornea based on corneal reflected light received by the light receiving element 266.

For example, the deformation detection system 260 may detect a deformation state of the cornea based on a magnitude of a light receiving signal of the light receiving element 266. For example, the deformation detection system 260 may detect that the cornea is in the applanation state when a light receiving amount of the light receiving element 266 is maximum. In this case, for example, the deformation detection system 260 is set in a manner that the light receiving amount becomes maximum when the cornea of the subject eye is in the applanation state.

The deformation detection system 260 may be an anterior segment cross-sectional image imaging unit such as an OCT or a Scheimplug camera. For example, the deformation detection system 260 may detect a deformation amount or a deformation speed of the cornea.

The Z alignment detection system 280 detects, for example, an alignment state in a Z direction. The Z alignment detection system 280 includes, for example, a light receiving lens 281 and a light receiving element 282. For example, the Z alignment detection system 280 may detect the alignment state in the Z direction by detecting reflected light from the cornea. For example, the Z alignment detection system may receive light that is emitted from the light source 261 and reflected by the cornea of the subject eye. In this case, the Z alignment detection system 280 may receive, for example, a bright point formed when light from the light source 261 is reflected by the cornea of the subject eye. In this way, the light source 261 may be used as a light source for Z alignment detection.

For example, light from the light source 261 reflected by the cornea passes through the beam splitter 204 and the light receiving lens 281 along the optical axis O4, and is received by the light receiving element 282. When the subject eye and the Z alignment detection system 280 are shifted in the Z direction, a light receiving position of the light from the light source 261 reflected by the cornea (for example, a position where an intensity of the light receiving signal is maximum) is shifted on the light receiving element 282. Therefore, the Z alignment detection system 280 may detect the alignment state based on the light receiving position of light from the light source 261 in the light receiving element 282. For example, the Z alignment detection system 280 may detect the alignment state by detecting whether the light receiving position of light from the light source 261 is a predetermined pixel (a detection reference position) of the light receiving element 282, or by detecting how many pixels are shifted from the predetermined pixel.

### <Control Unit>

Next, a configuration of a control system will be described with reference to FIG. 4. A control unit 70 performs, for example, control of the entire device, and arithmetic processing of a measurement value. The control unit 70 is implemented by, for example, a general central processing unit (CPU) 71, a ROM 72, and a RAM 73. The ROM 72 stores various programs, initial values, and the like for controlling operations of the ultrasonic ophthalmic tonometer 1. The RAM 73 temporarily stores various kinds of information. The control unit 70 may be implemented by one control unit or a plurality of control units (that is, a plurality of processors). The control unit 70 may be connected to, for example, the drive unit 5, a storage unit 74, a display unit 75, an operation unit 76, the irradiation unit 100, and the optical system 200.

The storage unit 74 is a non-transitory storage medium capable of storing storage contents even when a supply of power is cut off. For example, a hard disk drive, a flash ROM, or a detachable USB memory can be used as the storage unit 74.

The display unit 75 displays, for example, a measurement result of the subject eye. The display unit 75 may have a touch panel function.

The operation unit 76 receives various operation instructions from an inspector. The operation unit 76 outputs an operation signal corresponding to the input operation instruction to the control unit 70. At least one user interface such as a touch panel, a mouse, a joystick, or a keyboard may be used as the operation unit 76. When the display unit 75 is a touch panel, the display unit 75 may function as the operation unit 76.

### <Control Operation>

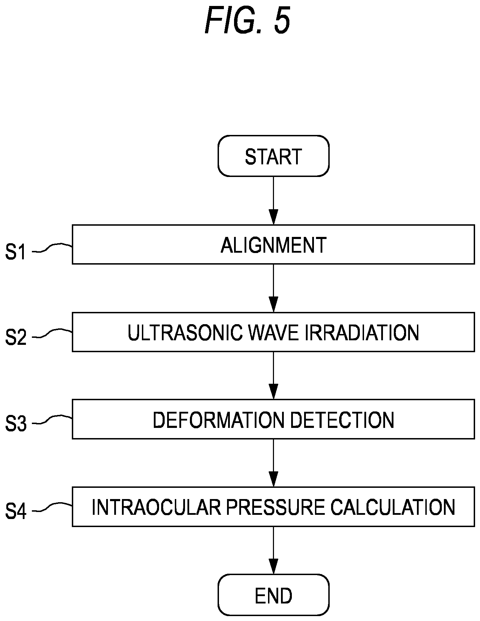

A control operation when measuring an intraocular pressure in the ultrasonic ophthalmic tonometer having the above configuration will be described with reference to FIG. 5.

### (Step S1: Alignment)

First, the control unit 70 performs alignment with respect to the subject eye of the subject whose face is supported by the face support unit 4. For example, the control unit 70 detects a bright point formed by an index projection system 250 from an anterior segment front image acquired by the light receiving element 222, and drives the drive unit 5 in a manner that the position of the bright point becomes a predetermined position. Of course, the inspector may manually perform alignment with respect to the subject eye by using the operation unit 76 or the like while viewing the display unit 75. When driving the drive unit 5, the control unit 70 determines whether alignment is appropriate based on whether the position of the bright point of the anterior segment image is the predetermined position.

### (Step S2: Ultrasonic Wave Irradiation)

The control unit 70 generates the ultrasonic wave by applying a voltage to the ultrasonic elements 110. The ultrasonic wave emitted from the irradiation unit 100 are applied to the subject eye, and the cornea of the subject eye deforms according to the acoustic radiation pressure of the ultrasonic wave.

### (Step S3: Deformation Detection)

The control unit 70 detects the deformation state of the cornea by the deformation detection system 260. For example, the control unit 70 detects that the cornea is deformed into the predetermined state (the applanation state or the flat state) based on the light receiving signal of the light receiving element 266.

### (Step S4: Intraocular Pressure Calculation)

For example, the control unit 70 calculates the intraocular pressure of the subject eye based on the acoustic radiation pressure (or the acoustic pressure) when the cornea of the subject eye is deformed into the predetermined state. The acoustic radiation pressure (or the acoustic pressure) applied to the subject eye correlates with the irradiation time of the ultrasonic wave, and increases as the irradiation time of the ultrasonic wave increases. Therefore, the control unit 70 obtains, based on the irradiation time of the ultrasonic wave, the acoustic radiation pressure (or the acoustic pressure) when the cornea is deformed into the predetermined state. The relation between the acoustic radiation pressure (or the acoustic pressure) when the cornea is deformed into the predetermined state and the intraocular pressure of the subject eye is obtained in advance by an experiment or the like and stored in the storage unit 74 or the like. The control unit 70 determines the intraocular pressure of the subject eye based on the acoustic radiation pressure (or the acoustic pressure) when the cornea is deformed into the predetermined state and the relation stored in the storage unit 74. In this way, the control unit 70 functions as a calculation unit that calculates the intraocular pressure.

Of course, the method for calculating the intraocular pressure is not limited to the above, and various methods may be used. For example, the control unit 70 may obtain a deformation amount of the cornea by using the deformation detection system 260, and may obtain the intraocular pressure by multiplying the deformation amount by a conversion factor. The control unit 70 may measure the intraocular pressure based on an ultrasonic wave reflected by the subject eye. For example, the intraocular pressure may be measured based on a characteristic change of the ultrasonic wave reflected by the subject eye, or the deformation amount of the cornea may be acquired from the ultrasonic wave reflected by the subject eye, and the intraocular pressure may be measured based on the deformation amount.

As described above, even in a case in which the parametric speaker is used as the irradiation unit 100, the ultrasonic ophthalmic tonometer 1 according to the present embodiment can appropriately dispose the irradiation unit 100 with respect to the subject eye by passing the optical axis O1 of the observation system 220 in the parametric speaker, and can observe the subject eye while irradiating the subject eye with the ultrasonic wave having a sufficient output.

In addition, by increasing the arrangement density of the ultrasonic elements 110 as in the above embodiment, an acoustic radiation pressure (or an acoustic pressure) necessary for intraocular pressure measurement can be generated while reducing the size of the irradiation unit 100.

The number and arrangement of the opening portions (or the holes) are not limited to the present embodiment. For example, only the central opening portion 102 may be provided with the support member 101. In this case, for example, the deformation detection system 260 and the Z alignment detection system 280 may be arranged in the front direction of the subject eye, and the optical axis O1 common to the observation system 220, the deformation detection system 260, and the Z alignment detection system 280 may pass through one opening portion 102. In addition to the three holes of the central opening portion 102 and the left and right holes 103 and 104, the support member 101 may be provided with a hole for an anterior segment illumination light source, an alignment bright point light source, or the like.

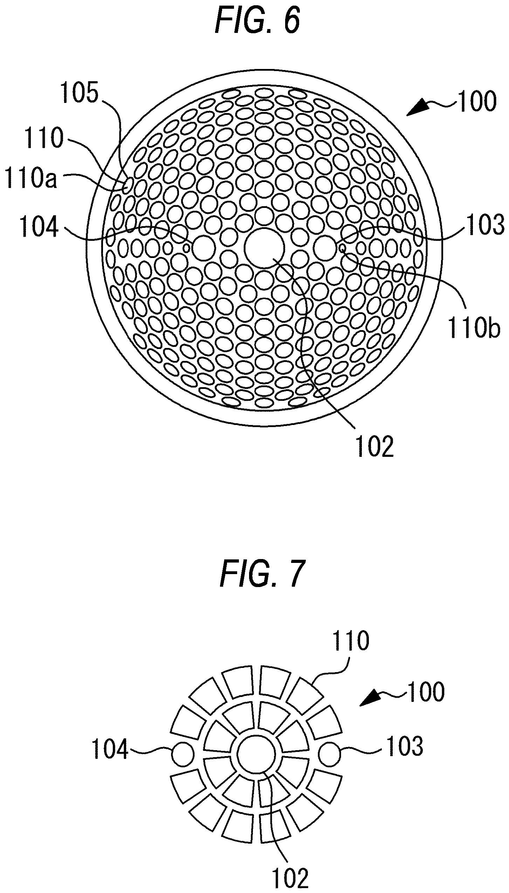

The size of each ultrasonic element 110 may be changed according to the arrangement. For example, as illustrated in FIG. 6, small ultrasonic elements 110b may be arranged in a gap when the ultrasonic elements 110 are arranged. Accordingly, wasted space can be reduced, and the sound source area can be increased. The shape of each ultrasonic element 110 (or a case housing the respective ultrasonic elements) may be changed according to the arrangement. For example, as illustrated in FIG. 7, the ultrasonic element 110 may have a fan shape and may be disposed in a manner of surrounding the opening portion 102. In this way, by changing the shape of the ultrasonic element 110, wasted space of the irradiation unit 100 can be reduced.

### REFERENCE SIGNS LIST

1 ultrasonic ophthalmic tonometer
2 base
3 housing
4 face support unit
5 drive unit
6 support group
100 irradiation unit
101 support member
102 opening portion
103 hole
104 hole
105 installation hole
200 optical system

## Claims

1. An ultrasonic ophthalmic tonometer that measures an intraocular pressure of a subject eye by using an ultrasonic wave, the ultrasonic ophthalmic tonometer comprising:
an imaging optical system configured to image the subject eye; and
an irradiation unit including a plurality of ultrasonic elements and a support member configured to support the ultrasonic elements, and irradiating the subject eye with an ultrasonic wave,
wherein an opening portion is provided with the support member, the opening portion configured to allow an optical axis of the imaging optical system to pass therethrough.

2. The ultrasonic ophthalmic tonometer according to claim 1,
wherein a diameter of each of the ultrasonic elements is smaller than a diameter of the opening portion.

3. The ultrasonic ophthalmic tonometer according to claim 1 or 2, further comprising:
a second optical system different from the imaging optical system,
wherein a light projecting system and a light receiving system of the second optical system are disposed obliquely with respect to the optical axis of the imaging optical system, and
a first hole and a second hole different from the opening portion are provided with the support member, the first hole configured to allow a light projecting optical axis of the second optical system to pass therethrough, the second hole configured to allow a light receiving optical axis of the second optical system to pass therethrough.

4. The ultrasonic ophthalmic tonometer according to any one of claims 1 to 3,
wherein a distance between centers of the plurality of ultrasonic elements is equal to or less than a wavelength of an ultrasonic wave to be emitted to the subject eye.

5. The ultrasonic ophthalmic tonometer according to any one of claims 1 to 4,
wherein the plurality of ultrasonic elements are arranged in a manner that sound source areas are symmetrical in left and right regions on the support member the and/or in upper and lower regions on the support member, the left and right regions provided in a case where the support member is divided by a vertical plane including the optical axis, the upper and lower regions provided in a case where the support member is divided by a horizontal plane including the optical axis.

6. The ultrasonic ophthalmic tonometer according to any one of claims 1 to 5, further comprising:
a deformation detection unit configured to detect that a cornea of the subject eye is deformed into a predetermined state by an ultrasonic wave from the irradiation unit; and
a calculation unit configured to calculate the intraocular pressure of the subject eye based on an ultrasonic wave output of the irradiation unit when the cornea is deformed into the predetermined state.
